(19) Europäisches Patentamt
European Patent Office
Office européen des brevets

(11) Publication number: **0 095 239**
**B1**

(12) # EUROPEAN PATENT SPECIFICATION

(45) Date of publication of patent specification: **09.07.86**

(51) Int. Cl.$^4$: **D 21 C 3/02,** C 07 D 209/08, C 12 P 17/10, C 12 P 17/16

(21) Application number: **83302018.3**

(22) Date of filing: **11.04.83**

(54) **Delignification of lignocellulosic material.**

(30) Priority: **30.04.82 GB 8212638**

(43) Date of publication of application:
**30.11.83 Bulletin 83/48**

(45) Publication of the grant of the patent:
**09.07.86 Bulletin 86/28**

(84) Designated Contracting States:
**AT DE FR GB SE**

(56) References cited:
**BULLETIN OF THE INSTITUTE OF PAPER CHEMISTRY, vol. 25, no. 10, June 1955, page 829, last paragraph; page 830, first paragraph, Appleton, Wisconsin, US T. ENKVIST et al.: "Biochemical experiments with spent liquors"**

**CHEM. PHARM. BULL., vol. 29, no. 4, April 1981, pages 961-969, Tokyo, JP K. ARAI et al.: "Metabolic products of aspergillus terreus. IV. Metabolites of the strain IFO 8835. (2). The isolation and chemical structure of indolyl benzoquinone pigments"**

(73) Proprietor: **IMPERIAL CHEMICAL INDUSTRIES PLC**
**Imperial Chemical House Millbank**
**London SW1P 3JF (GB)**

(72) Inventor: **Naylor, Thomas William**
**30 Grosvenor Road**
**Fairfield Stockton-on-Tees Cleveland (GB)**
Inventor: **Brown, Linda Anne**
**2 Formby Walk**
**Eaglescliffe Stockton-on-Tees Cleveland (GB)**

(74) Representative: **Aufflick, James Neil et al**
**Imperial Chemical Industries PLC**
**Legal Department: Patents**
**PO Box 6 Bessemer Road**
**Welwyn Garden City Herts, AL7 1HD (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 095 239 B1

**Description**

This invention relates to a process for the delignification of lignocellulosic material with an alkaline liquor in the presence of a quinonoid compound.

During the treatment of lignocellulosic material to produce cellulose suitable for the manufacture of paper products it is necessary to remove lignin and other non-cellulosic components such as gums. A number of processes for the treatment of lignocellulosic material are known and these processes involve cooking the material with alkaline liquors. Processes presently in use include:—

(a) The Kraft (sulphate) process wherein the lignocellulosic material is cooked with a mixture of sodium hydroxide and sodium sulphide;

(b) The Soda process wherein the lignocellulosic material is cooked with sodium hydroxide in the absence of sodium sulphide; and

(c) The two stage soda-oxygen pulping process described in Canadian Patent No. 895756.

These processes are effective in removing lignin from lignocellulosic material such as wood but in them the cellulose component on the material is attacked to some extent, resulting in lowered yields and in degradation of the product.

In the past decade there have been a number of proposals for improving the yields of processes for the delignification of lignocellulosic materials by use of quinonoid compounds. These proposals include:—

(1) Use of anthraquinone 2-monosulphonic acid (AMS) in the Soda process — B. Bach and G. Fiehn, Zellstoff Papier 21, 1, 3—7, January 1972 and related East German Patent 98549;

(2) Use of AMS in the first stage of the two stage soda-oxygen pulping process — US Patent 3888727; and

(3) Use of a defined group of cyclic keto compounds including naphthoquinone, anthraquinone and phenanthrenequinone in processes for the delignification of lignocellulosic materials generally — US Patent 4012280 assigned to Canadian Industries Limited.

The isolation from the fungus *Aspergillus terreus* IFO 6123 of a purple substituted benzoquinone has been reported by Y. Yamamoto et al (Chem. Pharm. Bull., *24*, 1853, (1976)). This compound is named by Yamamoto and his co-workers "asterriquinone" and its antitumour activity is described by them in Gann, *67*, 623, (1976). Analogous substituted benzoquinones having antitumour properties and derived from *Aspergillus terreus* var africanus IFO 8835 are described by K. Arai and Y. Yamamoto et al (Chem. Pharm. Bull., *29*(4), 961—969 (1981)) in an article which proposes that "asterriquinone" should be used as a general name for these compounds. K. Arai and Y. Yamamoto et al refer to these compounds as "pigments" and isolated them in the course of an investigation of antitumour compounds.

According to the present invention we provide a process for the delignification of lignocellulosic material which comprises the following steps:—

1. treating the lignocellulosic material, preferably in a closed reaction vessel, with an alkaline pulping liquor containing from 0.001% to 10.0% by weight, based on the lignocellulosic material, of a quinonoid additive material, the treatment taking place at a temperature within the range 150°C to 200°C for a period of 0.5 to 480 minutes, and

2. displacing the pulping liquor from the treated lignocellulosic material with water or an aqueous liquor inert to the lignocellulosic material;

wherein the quinonoid additive material is

(a) an indole substituted quinone having the formula

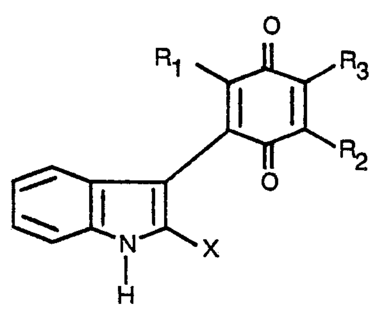

where X, $R_1$, $R_2$ and $R_3$ are hydrogen atoms, hydroxyl groups and/or organic substituents, or a microorganism, a cellular component, a crude or a refined extract of a microorganism containing said indole substituted quinone, and/or

(b) a microorganism, a cellular component, a crude or a refined extract of a microorganism which is a strain of *Aspergillus terreus* var *aureus*.

In the process of the invention, a wide variety of indole substituted quinones may be used as the quinoid additive material. Preferred organic substituents at $R_1$, $R_2$, $R_3$ and X are as follows:—

X — a saturated or unsaturated hydrocarbon group, particularly a substituted allyl group and especially a 3,3-dimethyl allyl group;

$R_1$ & $R_2$ — these substituents are preferably the same and are alkoxy-groups, particularly methoxy-groups; and

$R_3$ — an indole group.

A most suitable substituted quinone for use as the quinoid additive material in the delignification process of the invention is the substituted quinone (A)

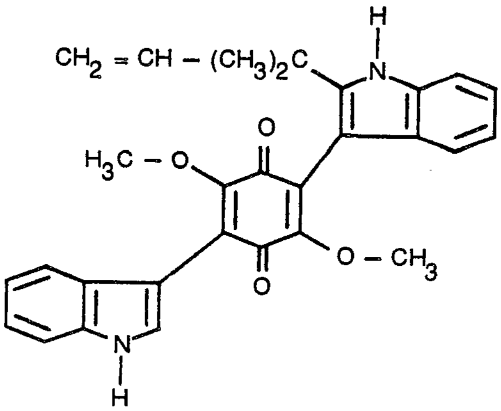

which contains a 5-membered ring substituted with a 3,3-dimethyl allyl group.

The alkaline pulping liquor used in step 1 of the process of the invention may contain anthraquinone in addition to the quinonoid additive material. When anthraquinone is present with the quinoid additive material in the pulping liquor it is preferred that the total amount of anthraquinone and quinonoid additive material is within the range 0.001% to 10% by weight based on the lignocellulosic material. Preferably the amount of anthraquinone is within the range of 20% to 80% (particularly 40% to 60%) by weight of the total weight of anthraquinone and quinonoid additive material.

In the process of the invention, the strain of the fungus *Aspergillus terreus* var *aureus* is preferably strain ATC 16793 — deposited at the American Type Culture Collection (ATCC), 12301 Parklawn Drive, Maryland 20852, USA.

The product produced by the two steps of the process of the invention is a substantially delignified cellulosic material which may be used without further treatment or which may be subjected to conventional bleaching steps.

The process of the invention may be used to treat any form of lignocellulosic material, such as hardwoods, bagasse, straw and in particular softwoods. When the lignocellulosic material is wood, it is suitably first converted into a suitable physical form, e.g. into chips. Soft woods which may be treated by the process of the invention include pine, spruce and fir. Whilst hardwoods to be treated include birch, beech and oak.

Any suitable alkaline pulping liquor may be used in the first step of the process of the invention. Preferably the liquor is one which is conventionally used, e.g. the liquor used in the conventional Kraft process or Soda process, with the quinonoid additive material added to it. The presence of the additive material enhances the yields and quality as judged by the kappa number of the resulting pulp.

Further information concerning alkaline pulping processes and in particular the Kraft Process as presently practiced is given in several texts for example in Kirk Othmer's Encyclopedia of Chemical Technology published by John Wiley & Son Inc., either 2nd Edition; Vol. 16, pp 702—712, (1968) or 3rd Edition; vol. 19, pp 395—408, (1982).

Soda process liquor suitably contains from 8% to 20% by weight of alkali metal base expressed as percent effective alkali, based on the weight of lignocellulosic material and normally also contains alkali metal carbonate.

Kraft process liquor suitably contains from 8% to 15% by weight of alkali metal base expressed as percent effective alkali and from 5% to 40% by weight of alkali metal sulphide, based on the weight of lignocellulosic material.

In the process of the invention the first treatment step is carried out at a temperature within the range 150°C to 200°C in the presence of water and preferably in a closed reaction vessel. Hence the first treatment step is usually carried out at above atmospheric pressure, e.g. at a gauge pressure of $7.24 \times 10^5$ Pa. After treatment with pulping liquor in the first treatment step, the resulting pulp yield is usually between 40% and 70% by weight, based on the lignocellulosic material. The kappa number of the material upon completion of the first step will therefore usually lie within the range 10 to 150 for coniferous woods and within the range 5 to 100 for deciduous woods. The substantially delignified material produced in the first treatment step is discharged from the pulping vessel, separated from the spent or black liquor and washed with water or a suitable aqueous stream. The spent 'black' liquor is used and inorganic materials in it are recycled as described in for example Kirk Othmer's Encyclopedia of Chemical Technology.

The alkali metal base used in the process of the invention is preferably sodium hydroxide, potassium hydroxide, sodium carbonate or potassium carbonate. The alkali metal sulphide is preferably sodium sulphide or potassium sulphide.

The quinonoid additive used in the process of the invention is suitably a substituted quinone obtained from strains of the genus Aspergillus, particularly strains of *Aspergillus terreus* var *aureus*, a most suitable strain of this species being ATCC 16793. When used in the process the substituted quinone may be present in pure form or included in a microorganism, a cellular component of a microorganism or in a crude or refined extract obtained from a microorganism or a cellular component.

To produce the substituted quinone, extract or compound including the substituted quinone to be used in the process of the invention, cells of a strain of the fungus *Aspergillus terreus* var *aureus* are cultured in any suitable culture medium using appropriate conditions. Suitably the culture medium may contain as a carbon source a sugar such as sucrose or glucose or a sugar alcohol. Preferred nitrogen sources are nitrates, ammonium ion or peptones or amino acids. Other preferred nutrients include phosphate and ferrous iron. Preferably cultivation takes place at a temperature within the range 10°C to 35°C, particularly 28°C and at a pH within the range pH 2 to pH 8, especially pH 6 to 7.5, a pH of 6.2 being particularly preferred.

The invention is illustrated by the following Examples:—

Example 1

Production of extracts from *Aspergillus terreus* var *aureus*

A spore suspension of the fungus *Aspergillus terreus* var *aureus* ATCC 16793 was prepared from an agar slant using Czapek Dox agar and incubated at 280°C to allow sporulation to occur. The Czapek Dox medium used had the following composition:—

| | |
|---|---|
| Sodium nitrate: | 2.00 g/l |
| Potassium chloride: | 0.50 g/l |
| Magnesium glycerophosphate: | 0.50 g/l |
| Ferrous sulphate: | 0.01 g/l |
| Potassium sulphate: | 0.35 g/l |
| Sucrose: | 30.00 g/l |

This medium was buffered to a pH within the range 2 to 7, typically pH 4.5.

The spores produced were then suspended in water and inoculated into culture vessels such as bottles or flasks which were incubated at temperatures within the range 10° to 35°C either statically or with shaking until product appeared after an interval varying from 5 to 10 days up to 28 days. This product was treated to produce an active product suitable for use in the process of the invention. In this treatment fungal mycelium was first separated from the culture and then dried. The resulting mycelium was ground to a powder and extracted in a soxhlet apparatus with petroleum ether for 4 hours. The petroleum ether soluble material was discarded. The mycelium was then re-extracted with methanol for approximately 5 hours. Evaporation of this methanol extract yielded a crude material which contained the active products. The active products were separated from the crude material by column or thin layer chromatography. This was done by fractionating in a column using chloroform and further purifying on thin layer plates or columns using toluene:ether (7.3) as solvent.

All the purified fractions and the crude methanol extract containing quinonoid materials were shown to be active in the process of the invention. The substituted quinone (A) whose structural formula is given hereinbefore was extracted from one of the purified fractions and was shown to be effective in the process of the invention.

Example 2

Example 1 was repeated except that the further purification of the product with toluene:ether was omitted. This further purification step was replaced by a treatment in which the dried chloroform extract was treated by addition of a non-polar solvent (preferably n-hexane). The substituted quinone (A) crystallized out and was separated by centrifugation or filtration.

Example 3

75 g of oven dried pine wood chips were used as the lignocellulosic material. The wood chips were soaked overnight in 229 ml of water. 61.5 ml of a 20% sodium hydroxide solution containing 4.0 g sodium sulphide, 300 mg of methanol extract (a crude extract obtained from *Aspergillus terreus* var *aureus* strain ATCC 16793) dissolved in 1 ml of methanol was added to the presoaked wood. The crude extract contained approximately 35 mg of the active quinonoid materials, that is .05% by weight, of the lignocellulosic material.

The wood was digested in a closed reaction vessel for 65 minutes at a temperature of 175°C. The pulp was washed with water to remove the spent liquor and % pulp yields and kappa numbers were determined.

The results are set out below in Table 1 and are compared with results obtained with a control using a treatment solution containing 1 ml methanol but without any crude extract.

TABLE 1

| Sample | % yield of dried cellulose related to dried wood | Kappa No. |
|---|---|---|
| Control | 43.4 | 43.9 |
| Sample using treatment solution containing crude extract | 46.7 | 40.5 |

The results show an improvement in yield and kappa number for the sample treated with solution including crude extract.

Example 4

Example 3 was repeated except that in the treatment solution according to the invention the 300 mg of crude extract dissolved in 1 ml of methanol was replaced by 37.5 mg of the substituted quinone (A) dissolved in 1 ml of methanol. The results are set out in Table 2 and again indicate improvements in yield and kappa number for the sample treated according to the invention.

TABLE 2

| Sample | % yield of dried cellulose related to dried wood | Kappa No. |
|---|---|---|
| Control | 43.9 | 43.4 |
| Sample using treatment solution containing substituted quinone (A) | 45.7 | 41.1 |

Example 5

Example 4 was repeated but in this instance the substituted quinone (A) was tested in combination with anthraquinone. Anthraquinone was also tested in the absence of the substituted quinone.

It was shown that anthraquinone and the substituted quinone were compatible in the wood pulping tests and that the individual effects of the substances were both present in the mixtures. Anthraquinone reduces kappa number but does not affect yield in the conditions of these tests. The substituted quinone (A) increases yield with little effect on kappa number in similar tests. When both are used together yield and kappa number effects are both observed. The test with both anthraquinone and substituted quinone (A) was repeated to show that the result was reproducible. The results are summarised in Table 3.

TABLE 3

| Sample | % yield of dried cellulose related to dried wood | Kappa No. |
|---|---|---|
| Control | 47.1 | 39.1 |
| Anthraquinone (0.05%) | 47.2 | 35.8 |
| Anthraquinone (0.025%) + Substituted quinone (0.025%) | 48.3 | 36.8 |
| Anthraquinone (0.025%) + Substituted quinone (0.025%) | 48.6 | 36.8 |

**Claims**

1. A process for the delignification of lignocellulosic material which comprises the following steps:—

(1) treating the lignocellulosic material with an alkaline pulping liquor containing from 0.001% to 10.0% by weight, based on the lignocellulosic material, of a quinonoid additive material, the treatment taking place at a temperature within the range 150°C to 200°C for a period of 0.5 to 480 minutes, and

(2) displacing the pulping liquor from the treated lignocellulosic material with water or an aqueous liquor inert to the lignocellulosic material; wherein the quinonoid additive material is

(a) an indole substituted quinone having the formula

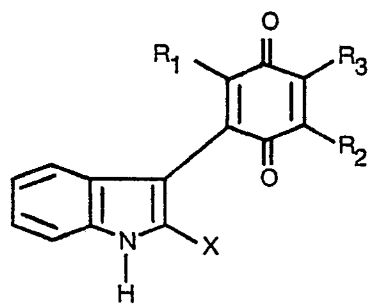

where X, $R_1$, $R_2$ and $R_3$ are hydrogen atoms, hydroxyl groups and/or organic substituents, or a microorganism, a cellular component, a crude or a refined extract of a microorganism containing said indole substituted quinone, and/or

(b) a microorganism, a cellular component, a crude or a refined extract of a microorganism which is a strain of *Aspergillus terreus* var *aureus*.

2. A process according to claim 1 wherein step (1) is carried out in a closed reaction vessel.

3. A process according to claim 1 or claim 2 wherein in the formula of the indole substituted quinone X is an unsaturated hydrocarbon group.

4. A process according to claim 3 wherein the unsaturated hydrocarbon group is a substituted allyl group.

5. A process according to any one of the preceding claims wherein in the formula of the indole substituted quinone $R_1$ and $R_2$ are the same and are alkoxy groups.

6. A process according to claim 5 wherein $R_1$ and $R_2$ are methoxy groups.

7. A process according to any one of the preceding claims wherein in the formula of the indole substituted quinone $R_3$ is an indole group.

8. A process according to claim 1 wherein the indole substituted quinone has the formula

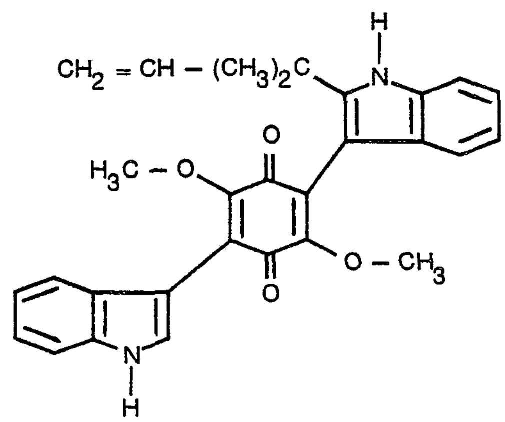

9. A process according to any one of the preceding claims wherein the alkaline pulping liquor contains anthraquinone in addition to the quinonoid additive material.

10. A process according to claim 9 wherein the total amount of anthraquinone and quinonoid additive material in the alkaline pulping liquor is from 0.001% to 10.0% by weight based on the lignocellulosic material and the amount of anthraquinone present is within the range 40% to 60% by weight of the total weight of anthraquinone and quinonoid additive material.

11. A process according to any one of the preceding claims wherein the strain of *Aspergillus terreus* var *aureus* is strain ATCC 16793.

12. A process according to any one of the preceding claims wherein the lignocellulosic material is a softwood.

13. A process according to any one of the preceding claims wherein the lignocellulosic material is wood converted into chips.

14. A process according to any one of the preceding claims wherein the alkaline pulping liquor is a Soda process liquor containing 8% to 20% by weight of alkali metal base expressed as percent effective alkali based on the weight of lignocellulosic material.

15. A process according to any one of claims 1 to 13 wherein the alkaline pulping liquor is a Kraft process liquor containing 8% to 15% by weight of alkali metal base expressed as percent effective alkali and 5% to 40% by weight of alkali metal sulphide based on the weight of lignocellulosic material.

**Patentansprüche**

1. Verfahren zur Delignifizierung von Holzcellulosematerial mit den folgenden Schritten:

(1) Behandlung des Holzcellulosematerials mit einer alkalischen Aufschlußlösung, die 0,001 bis 10,0 Gew.-%, bezogen auf das Holzcellulosematerial, eines chinoiden Zusatzstoffes enthält, wobei die Behandlung 0,5 bis 480 min lang bei einer Temperatur in dem Bereich von 150°C bis 200°C stattfindet, und

(2) Verdrängung der Aufschlußlösung aus dem behandelten Holzcellulosematerial mit Wasser oder mit einer genenüber dem Holzcellulosematerial inerten wäßrigen Flüssigkeit; wobei der chinoide Zusatzstoff

(a) ein indolsubstituiertes Chinon mit der Formel:

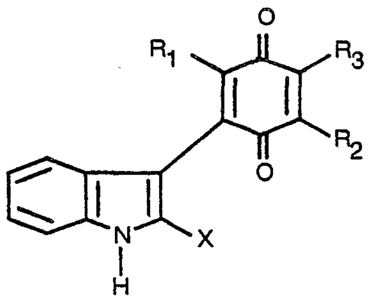

worin X, $R_1$, $R_2$ und $R_3$ Wasserstoffatome, Hydroxylgruppen und/oder organische Substituenten sind, oder ein Mikroorganismus, ein Zellbestandteil, ein Rohextrakt oder ein gereinigter Extrakt eines Mikroorganismus, der das erwähnte indolsubstituierte Chinon enthält, und/oder

(b) ein Mikroorganismus, ein Zellbestandteil, ein Rohextrakt oder ein gereinigter Extrakt eines Mikroorganismus ist, der ein Stamm von *Aspergillus terreus* var *aureus* ist.

2. Verfahren nach Anspruch 1, bei dem Schritt (1) in einem geschlossenen Reaktionsbehälter durchgeführt wird.

3. Verfahren nach Anspruch 1 oder 2, bei dem X in der Formel des indolsubstituierten Chinons eine ungesättigte Kohlenwasserstoffgruppe ist.

4. Verfahren nach Anspruch 3, bei dem die ungesättigte Kohlenwasserstoffgruppe eine substituierte Allylgruppe ist.

5. Verfahren nach einem der vorhergehenden Ansprüche, bei dem $R_1$ und $R_2$ in der Formel des indolsubstituierten Chinons gleich sind und Alkoxygruppen bedeuten.

6. Verfahren nach Anspruch 5, bei dem $R_1$ und $R_2$ Methoxygruppen bedeuten.

7. Verfahren nach einem der vorhergehenden Ansprüche, bei dem $R_3$ in der Formel des indolsubstituierten Chinons eine Indolgruppe ist.

8. Verfahren nach Anspruch 1, bei dem das indolsubstituierten Chinon die folgende Formel hat:

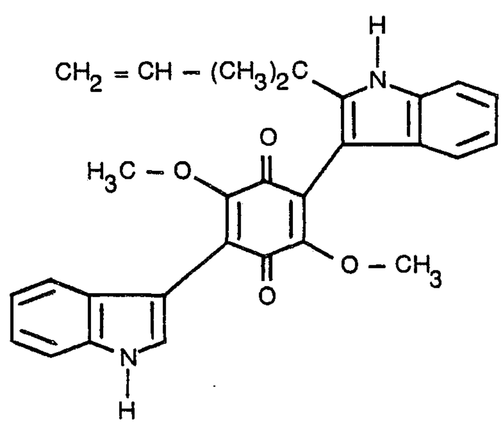

9. Verfahren nach einem der vorhergehenden Ansprüche, bei dem die alkalische Aufschlußlösung zusätzlich zu dem chinoiden Zusatzstoff Anthrachinon enthält.

10. Verfahren nach Anspruch 9, bei dem die Gesamtmenge an Anrhrachinon und chinoidem Zusatzstoff in der alkalischen Aufschlußlösung 0,001 bis 10,0 Gew.-%, bezogen auf das Holzcellulosematerial, beträgt und die Menge des vorhandenen Anthrachinons in dem Bereich von 40 bis 60 Gew.-%, bezogen auf das Gesamtgewicht des Anthrachinons und des chinoiden Zusatzstoffs, liegt.

11. Verfahren nach einem der vorhergehenden Ansprüche, bei dem der Stamm von *Aspergillus terreus* var *aureus* der Stamm ATCC 16793 ist.

12. Verfahren nach einem der vorhergehenden Ansprüche, bei dem das Holzcellulosematerial ein Nadelholz bzw. Weichholz ist.

13. Verfahren nach einem der vorhergehenden Ansprüche, bei dem das Holzcellulosematerial in Hackspäne umgewandteltes Holz ist.

14. Verfahren nach einem der vorhergehenden Ansprüche, bei dem die alkalische Aufschlußlösung eine Sodaverfahren-Lösung ist, die 8 bis 20 Gew-% Alkalimetallbase, als Prozent effektives Alkali ausgedrückt, bezogen auf das Gewicht des Holzcellulosematerials, enthält.

15. Verfahren nach einem der Ansprüche 1 bis 13, bei dem die alkalische Aufschlußlösung eine Sulfatverfahren-Lösung ist, die 8 bis 15 Gew.-% Alkalimetallbase, als Prozent effektives Alkali ausgedrückt, und 5 bis 40 Gew.-% Alkalimetallsulfid, bezogen auf das Gewicht des Holzcellulosematerials, enthält.

**Revendications**

1. Procédé pour la délignification de matière lignocellulosique qui comprend les étapes suivantes:

(1) on traite la matière lignocellulosique avec une liqueur alcaline de lessivage contenant de 0,001 à 10,0% en poids d'un additif quinonoïde par rapport à la matière lignocellulosique, à une température dans la gamme de 150° à 200°C pendant une période de 0,5 à 480 minutes, et

(2) on déplace la liqueur de lessivage à partir de la matière lignocellulosique traitée avec de l'eau ou une liqueur aqueuse inerte vis-à-vis de la matière lignocellulosique; caractérisé en ce que l'additif quinonoïde est

(a) une quinone substituée par un groupe indole, représentée par la formule

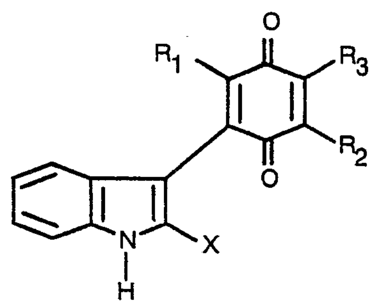

dans laquelle X, $R_1$, $R_2$ et $R_3$ sont des atomes d'hydrogène, des groupes hydroxy et/ou des substituants organiques, ou un microorganisme, un composant cellulaire, un extrait brut ou raffiné d'un microorganisme contenant cette quinone substituée par le groupe indole, et/ou

(b) un microorganisme, un composant cellulaire, un extrait brut ou raffiné d'un microorganisme qui est une souche de *Aspergillus terreus* var *aureus*.

2. Procédé suivant la revendication 1, caractérisé en ce que l'étape (1) est effectuée dans un réacteur fermé.

3. Procédé suivant la revendication 1 ou la revendication 2, caractérisé en ce que dans la formule de la quinone substituée par un groupe indole, X représente un groupe hydrocarboné insaturé.

4. Procédé suivant la revendication 3, caractérisé en ce que le groupe hydrocarboné insaturé est un groupe allyle substitué.

5. Procédé suivant l'une quelconque des revendications 1 à 4, caractérisé en ce que dans la formule de la quinone substituée par un groupe indole, $R_1$ et $R_2$ sont identiques et représentent des groupes alcoxy.

6. Procédé suivant la revendication 5, caractérisé en ce que $R_1$ et $R_2$ sont des groupes méthoxy.

7. Procédé suivant l'une quelconque des revendications 1 à 6, caractérisé en ce que dans la formule de la quinone substituée par un groupe indole, $R_3$ est un groupe indole.

8. Procédé suivant la revendication 1, caractérisé en ce que la quinone substituée par un groupe indole présente la formule:

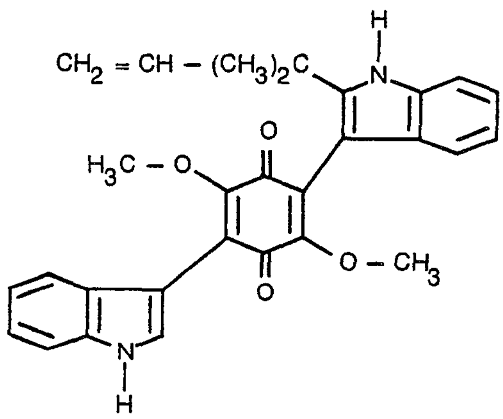

9. Procédé suivant l'une quelconque des revendications 1 à 8, caractérisé en ce que la liqueur alcaline de lessivage contient de l'anthraquinone en plus de l'additif quinonoïde.

10. Procédé suivant la revendication 9, caractérisé en ce que la quantité totale d'anthraquinone et d'additif quinonoïde dans la liqueur alcaline de lessivage, est de 0,001% à 10% en poids par rapport à la matière lignocellulosique et que la quantité d'anthraquinone présente est dans la gamme de 40 à 60% en poids par rapport au poids total d'anthraquinone et d'additif quinonoïde.

11. Procédé suivant l'une quelconque des revendications 1 à 10, caractérisé en ce que la souche d'*Aspergillus terreus* var *aureus* est la souche ATCC 16793.

12. Procédé suivant l'une quelconque des revendications 1 à 11, caractérisé en ce que la matière lignocellulosique est un bois tendre.

13. Procédé suivant l'une quelconque des revendications 1 à 12, caractérisé en ce que la matière lignocellulosique est du bois mis sous forme de copeaux.

14. Procédé suivant l'une quelconque des revendications 1 à 13, caractérisé en ce que la liqueur alcaline de lessivage est une liqueur du procédé à la soude, contenant 8% à 20% en poids d'une base de métal alcalin, exprimés en % d'alcali efficace, par rapport au poids de la matière lignocellulosique.

15. Procédé suivant l'une quelconque des revendications 1 à 13, caractérisé en ce que la liqueur alcaline de lessivage est une liqueur du procédé Kraft contenant 8% à 15% en poids d'une base de métal alcalin, exprimés en % d'alcali efficace, et 5% à 40% en poids de sulfure de métal alcalin par rapport au poids de la matière lignocellulosique.